# EUROPEAN PATENT APPLICATION

(11) **EP 4 012 038 A1**
(43) Date of publication of application: **15.06.2022**
(21) Application number: 20213385.6
(22) Date of filing: 11.12.2020
(51) Int. Cl.: C12P 7/10, C12P 19/14, C08H 8/00

(54) **METHOD FOR CONTROLLED PRETREATMENT AND/OR ENZYMATIC HYDROLYSIS OF A LIGNOCELLULOSIC MATERIAL**

(71) Applicant: Sekab E-Technology AB, 891 26 Örnsköldsvik (SE)
(72) Inventor: CAVKA, Adnan, 891 96 Arnäsvall (SE); SUNDVALL, Elias, 892 32 Domsjö (SE)
(74) Representative: Kransell & Wennborg KB

(57) **Abstract**

The present disclosure generally relates to a controlled method for pretreatment and/or enzymatic hydrolysis of a lignocellulosic material. The method comprises first pretreating the lignocellulosic material suboptimally, and then subjecting the material to mechanical refining.

## Description

### Technical field

The present disclosure generally relates to a method for controlled pretreatment and/or enzymatic hydrolysis of a lignocellulosic material. The method comprises first pretreating the lignocellulosic material suboptimally, and then subjecting the material to mechanical refining.

### Background

Lignocellulosic residues from forestry are attractive as feedstocks for the production of renewable chemicals and fuels, since they are abundant, relatively inexpensive, and are not used for food. Lignocellulose consists mainly of lignin and two classes of polysaccharides, cellulose and hemicellulose. The polysaccharides can be hydrolyzed to sugars and thereafter converted to various fermentation products, e.g. bio-alcohols, by means of microorganisms, such as baker's yeast *Saccharomyces cerevisiae.*

During enzymatic hydrolysis of lignocellulosic materials, the cellulose present is partly converted into fermentable sugars by cellulolytic enzymes.

The hydrolysis of cellulose is typically preceded by a pretreatment process, in which the hemicellulose is degraded, and the cellulose is made increasingly accessible to the enzymes.

The pretreatment process is considered to be one of the most critical parts in the process of converting biomass into fermentation products, mainly because it affects downstream processes and strongly impacts the overall sugar yields.

A variety of pretreatment processes exist, many of which rely on elevated temperatures and pressures, generally including catalytic agents. Often, the pretreatment process conditions need to be relatively severe or "harsh" to increase the enzymatic availability during subsequent hydrolysis.

While conventional processes typically focus on maximizing the sugar yield and optimizing the pretreatment to enhance the conversion of cellulose into sugar, it should be noted that a too severe pretreatment may lead to degradation of sugars and increased levels of substances which are toxic to hydrolytic enzymes and/or fermenting organisms. Such substances may be referred to as hydrolysis or fermentation inhibitors, and include e.g. furfural, hydroxymethylfurfural, acetic acids and phenolic compounds.

Furthermore, a too harsh pretreatment may give rise to the formation of pseudo-lignin. Pseudo-lignin is a lignin like and acid-insoluble material consisting of carbonyl, carboxylic, aromatic and aliphatic structures, which may form a coating on the surface of the pretreated biomass particles. Such a pseudo-lignin coating may impair subsequent saccharification by preventing the enzyme access to the carbohydrates of the biomass during enzymatic hydrolysis.

A too harsh pretreatment may also pose demands on the equipment utilized for the pretreatment process. For example, high temperatures and pressures may result in the accumulation of solid material and deposits in the pretreatment reactor.

It is generally difficult to find a streamlined optimal pretreatment, since the process conditions vary significantly between different lignocellulosic starting materials. This is mainly due to the differences in composition and structural characteristics of the lignocellulose material. The chemical or physical nature of the ingoing lignocellulosic material may vary greatly. For example, the pretreatment process requirements for softwood are very different from those for hardwood, and large variations within these general groups also exist.

Hence, there is a need for a more controlled pretreatment and hydrolysis process, wherein the process conditions for different lignocellulosic starting materials are taken into account and optimized. Such a process should also be gentle and serve to reduce the formation of toxic by-products during pretreatment.

### Summary

In view of the above, it is a general object of the present disclosure to provide improvements with respect to controlling the pretreatment and/or enzymatic hydrolysis in order to reduce the formation of hydrolysis and fermentation inhibitors, pseudo-lignin as well as deposits in the equipment utilized.

According to a first aspect of the present disclosure, there is provided a method for controlled pretreatment and/or enzymatic hydrolysis of a lignocellulosic material comprising:
a) selecting a lignocellulosic material to be pretreated,
b) subjecting the lignocellulosic material to a suboptimal pretreatment process to form a first, suboptimally pretreated lignocellulosic composition,
c) subjecting the first, suboptimally pretreated lignocellulosic composition to mechanical refining to form a second lignocellulosic composition comprising lignocellulosic particles, and
d) subjecting the second lignocellulosic composition to enzymatic hydrolysis, wherein the suboptimal pretreatment process is a pretreatment process, which after enzymatic hydrolysis generates a cellulose-to-sugar conversion yield of less than 50% of the maximum theoretical cellulose-to-sugar conversion yield for the selected lignocellulosic material.

In contrast to conventional pretreatment and hydrolysis processes, where optimization of all process parameters is the goal to increase the sugar yield during hydrolysis, the present inventors have found that a suboptimal pretreatment process is desirable. A suboptimal pretreatment yields a lower amount of toxic substances and a significantly reduced amount of pseudo-lignin. Furthermore, a suboptimal pretreatment reduces fouling; i.e. accumulation of solid material and deposits in the pretreatment reactor.

A "suboptimal pretreatment process" is a process, which after enzymatic hydrolysis generates a cellulose-to-sugar conversion yield of less than 50% of the maximum theoretical cellulose-to-sugar conversion yield for the selected lignocellulosic material. The term "after enzymatic hydrolysis" means the yield obtained when hydrolysis is performed directly after the pretreatment process (i.e. not including the mechanical refining step).

In contrast, an "optimal pretreatment process" is a process, which after enzymatic hydrolysis generates a cellulose-to-sugar conversion yield of more than 50%, preferably more than 60% of the maximum theoretical cellulose-to-sugar conversion yield for the selected lignocellulosic material

The "maximum theoretical cellulose-to sugar conversion yield" depends on the chemical composition of the lignocellulosic material to be pretreated. The maximum theoretical cellulose-to-sugar conversion yield can be calculated for a particular lignocellulosic material by first determining the polysaccharide content, individual cellulose, glucomannan, and xylan content. For example, if the lignocellulosic material has a glucan content of 40 gram, the maximum theoretical yield of total glucose will be 40/0.9. If the lignocellulosic material has a xylan content of 20 grams, the maximum theoretical yield of total xylose will be 20/0.88.

The inventors have found that the conditions for pretreating and hydrolyzing various types of lignocellulosic materials vary greatly, and the awareness and knowledge of the conditions for a specific starting material can be utilized to intentionally pretreat that material suboptimally. The parameters of the pretreatment process may be adjusted to yield a low cellulose-to-sugar conversion yield for the purpose of reducing the amount of pseudo-lignin of the polysaccharides of the pretreatment slurry and for overcoming problems with fouling.

The suboptimal pretreatment process is thereafter compensated by subjecting the first, suboptimally pretreated lignocellulosic composition to mechanical refining to form a second pretreated composition comprising lignocellulosic particles. The mechanical refining may involve milling or grinding.

In this step, the larger particles and components of the first, suboptimally pretreated lignocellulosic composition are reduced in size, and the enzymatic availability for subsequent hydrolysis is improved.

The step of mechanical refining reduces the particle size and increases the available specific surface area for subsequent hydrolysis. The increased surface area makes the cellulosic material more accessible for the saccharification enzymes.

Mechanical refining may be performed by means of any type of mechanical refiner that reduces the size of the components of the suboptimally pretreated lignocellulosic composition. For example, the mechanical refiner may be any suitable type of mill or a single or double disc refiner. Preferably, the mechanical refiner is a disc refiner.

The mechanical refining step improves the sugar yield and results in a lower dosage of enzymes required in the subsequent hydrolysis step. Without wishing to be bound by theory, it is believed that the mechanical refining serves to break up and remove any potential pseudo-lignin that has been formed during pretreatment and that may be blocking the saccharification enzymes to access the surface of the lignocellulosic particles.

The term "sugar yield" means the fraction of sugar monomers and following hydrolysis relative to the lignocellulose material entering the hydrolysis after the pretreatment and mechanical refining steps.

The lignocellulosic material may be, but is not limited to hardwoods, softwoods, sugarcane bagasse, energy cane, corn stover, corn cobs, corn fibers, straw from rice, wheat, rye and other crops residues.

After mechanical refining, the second pretreated composition is subjected to enzymatic hydrolysis.

The hydrolysis reaction is catalyzed by at least one saccharification enzyme. A "saccharification enzyme" refers to at least one enzyme that can convert or hydrolyze (ligno) cellulosic material into fermentable saccharides, such as monosaccharides and/or disaccharides. Such saccharification enzymes may be glycosidases, which hydrolyze polysaccharides. Examples of glycosidases include cellulose-hydrolyzing glycosidases, such as cellulases, endoglucanases, exoglucanases, cellobiohydrolases and β-glucosidases, hemicellulose hydrolysing glycosidases, such as xylanases, endoxylanases, exoxylanases, β-xylosidases, arabinoxylanases, mannanases, galactanases, pectinases and glucuronases, and, or any enzymes in the group of enzymes found in EC 3.2.1.x, such as EC 3.2.1.4, where EC is the Enzyme Commission number.

The hydrolysate formed in the process of the present disclosure may comprise sugars from lignocellulosic biomass, such as glucose, mannose, xylose, arabinose, galactose, and sucrose.

In embodiments, the hydrolysate comprises at least glucose. In alternative embodiments, the hydrolysate comprises at least two different sugars.

In embodiments, at least 30%, e.g. at least 35%, e.g. at least 50%, e.g. at least 70% of the lignocellulosic particles of the first, suboptimally pretreated lignocellulosic composition has an average diameter above 500 µm, such as above 600 µm. In embodiments, for instance where the ingoing lignocellulosic material is spruce sawdust, the average diameter is above 700 µm, such as above 800 µm.

In other words, the particles formed after pretreatment are relatively large, and the compact structure of the first, suboptimally pretreated composition has only partially been disrupted.

The method may further comprise the step of evaluating the optimal process parameters for the selected lignocellulosic material to be pretreated prior to the suboptimal pretreatment step b).

As used herein, the term "optimal process parameters" or "optimal process parameter range" refers to the ideal range of one or several specific parameter that affects the pretreatment process by increasing the enzyme availability and maximizing the sugar yield in a subsequent hydrolysis process. The "optimal process parameter range" is specific to one lignocellulosic material. For example, an "optimal process parameter range" for one lignocellulosic material will not be "optimal" for another lignocellulosic material. Such optimal ranges may be the result of knowledge and experience gained from a vast number of pretreatment trials for a vast number of lignocellulosic materials. In this regard, it should be noted that lignocellulosic materials differ widely in nature, composition and structure, even within a specific lignocellulosic material category.

In embodiments, the suboptimal pretreatment process involves pretreating the lignocellulosic material in at least one suboptimal process parameter range, outside of an optimal process parameter range for the selected lignocellulosic material.

A "suboptimal process parameter range" is a range which, after hydrolysis, yields a low cellulose-to-sugar conversion yield; i.e. less than 50%, e.g. less than 30%, e.g. less than 25% of the theoretical cellulose-to-sugar conversion yield for a specific lignocellulosic material. A "suboptimal process parameter range" is typically at least 5% lower, e.g. at least 10% lower than the "optimal process parameter range". The suboptimal process parameter range is typically lower than the optimal process parameter range.

In embodiments, the suboptimal or optimal process parameter is the temperature, pressure or residence time in the pretreatment unit, and wherein the at least one suboptimal process parameter range is at least 5%, preferably at least 10% lower than the optimal process parameter range.

However, in the case of pH, the pH range may actually be higher than the optimal pH range in order to yield a suboptimally pretreated lignocellulosic composition. If the suboptimal or optimal process parameter is the pH, the pH may be at least 5%, preferably at least 10% higher than the optimal pH range.

In a method according to the present disclosure, a lignocellulosic material to be pretreated and hydrolyzed is first selected. Then, at least one optimal process parameter range for the selected lignocellulosic material is evaluated. The suboptimal pretreatment process involves pretreating the lignocellulosic material by deliberately selecting a parameter range outside of the optimal parameter range such that a suboptimally pretreated lignocellulosic composition is obtained.

The suboptimal or optimal process parameter may be selected from pH, temperature, residence time, and pressure of the pretreatment.

In embodiments, the suboptimal or optimal process parameter is the temperature. The temperature has a high impact on the degree of pretreatment. By selecting a lower temperature range, a significantly reduced amount of undesired by-products, including i.a. hydrolysis and fermentation inhibitors, and pseudo-lignin is formed. Furthermore, a milder pretreatment is achieved.

For example, if spruce sawdust is selected as the starting material, and the optimal temperature range is from 195 to 205°C, the suboptimal pretreatment may involve pretreatment at a temperature in a lower temperature range, such as between 175 and 185°C.

As another illustrative example, if straw is selected as the lignocellulosic starting material, and its optimal temperature range is from 175 to 185°C, the suboptimal pretreatment may involve pretreating at a temperature in a range of from 156 to 166°C.

In embodiments, at least two suboptimal process parameter ranges are involved in the suboptimal pretreatment; the suboptimal process parameters being selected from a pH, temperature, pressure or residence time in the pretreatment unit.

This is to even further reduce the severity of the pretreatment step and to reduce the amount of pseudo-lignin formed.

In embodiments, the suboptimal pretreatment process is a pretreatment process, which after enzymatic hydrolysis generates a cellulose-to sugar conversion yield of less than 30 % of the maximum theoretical cellulose-to sugar conversion yield for the selected lignocellulosic material. For example, the cellulose-to sugar conversion yield may be less than 25 %, e.g. less than 20 % of the theoretical cellulose-to sugar conversion yield for the selected lignocellulosic material.

In embodiments, the content of suspended solids of the suboptimally pretreated lignocellulosic composition is in the range of from 2 to 20%.

Any process for pretreating the lignocellulosic material may be used as long as the process parameters are controlled and monitored to yield a suboptimally pretreated lignocellulosic composition. The pretreatment can be made by any hydrothermal/chemical, physical or biological pretreatment methods.

For example, the pretreatment may involve acid pretreatment, alkali pretreatment, ammonia fiber explosion, acid catalyzed nor non catalyzed steam explosion, autohydrolysis, sulfite pulping, solvolysis, such as acid solvolysis. Further, the pretreatment may include impregnation, which refers to absorption or mass transfer of an impregnation fluid or gas to the lignocellulosic material, followed by above exemplified processing and/or pretreatment. The fluid may e.g. be solvents or solutions of acids such as sulphurous acid, sulphuric acid, ammonia, sulfite/bisulfite, sulfate hydroxides, and alcohols. The impregnation may also be performed using gas, such as a SO₂-gas or CO₂- gas, or with the combination of a gas and fluid of any given combination, concentration and proportion. The pretreatment may also comprise steaming. Steaming refers to a process used to drive air out from the cellulosic biomass to facilitate further hydrolysis of the cellulose. Steaming is a well-known method for pretreating e.g. lignocellulosic biomass.

In embodiments, the suboptimal pretreatment comprises steam explosion.

During steam explosion, hot steam is used to increase the pressure in the reactor and to disrupt of bonding between polymeric components of the lignocellulosic biomass. Thereafter, decompression from an increased pressure to atmospheric pressure occurs, which results in that the lignocellulose biomass is broken up into smaller particle sizes.

As an illustrative example, a suboptimal steam explosion may involve treating the lignocellulosic composition with hot steam at a pressure of about 13 to 16 bar (g) for about 5 to 45 minutes. However, as mentioned hereinbefore, the suboptimal pretreatment varies greatly depending on the type of and characteristic of the ingoing lignocellulosic material. The range specified above may be e.g. suitable for sawdust and wood chips, but may not be suitable for other types or lignocellulose materials.

The subsequent mechanical refining step (step c) yields a second lignocellulosic composition comprising lignocellulosic particles of a significantly smaller average size.

In embodiments, at least 50%, such as at least 70%, preferably at least 80% of the lignocellulosic particles of the second pretreated composition has an average diameter in the range of from 0.1 to 500 µm. For example, the average diameter may be in the range of from 0.1 to 200 µm, e.g. in the range of from 0.1 to 100 µm. The small size of the particles yields an improved sugar yield in the subsequent hydrolysis step.

According to an aspect of the present disclosure, there is provided a method for controlled pretreatment and/or enzymatic hydrolysis of a lignocellulosic material comprising:
a) selecting a lignocellulosic material to be pretreated,
b) evaluating at least one optimal process parameter range for pretreating said selected lignocellulosic material,
c) pretreating said lignocellulosic material in a process involving at least one suboptimal process parameter range, outside of said at least one optimal process parameter range to form a first, suboptimally pretreated lignocellulosic composition,
d) subjecting said first, suboptimally pretreated lignocellulosic composition to mechanical refining to form a second lignocellulosic composition comprising lignocellulosic particles, and
e) subjecting said second pretreated composition to enzymatic hydrolysis.

In embodiments, the method of the present disclosure further comprises the steps of:
- analyzing the first, suboptimally pretreated lignocellulosic composition to obtain a data set reflecting the degree of the pretreatment,
- controlling the mechanical refining in response to the data set.

By incorporating an analysis step after the pretreatment, the mechanical refining can be controlled, which allows for a more controlled and sophisticated pretreatment and hydrolysis process.

Lignocellulosic materials differ widely in nature, composition, structure etc., and various pretreatment methods also vary considerably. The step of analyzing the first, suboptimally pretreated lignocellulosic composition to obtain a data set reflecting the degree of the pretreatment may therefore yield valuable insight towards how to optimize the biomass treatment with the ultimate goal to increase the sugar yield, minimize enzyme consumption and obtaining an energy- and cost-efficient process.

This way, the mechanical refining can be controlled; i.e. by adjusting the mechanical refining in response to the data set. Furthermore, the method allows for a more controlled, and situation-adapted pretreatment and hydrolysis which enables swift adjustments in view of the characteristics of the lignocellulosic biomass after pretreatment. Factors such as degree of pretreatment, composition, chemical and physical nature of the lignocellulosic starting material etc. may therefore be taken into account. Depending on the properties and characteristics of the components of the first, suboptimally pretreated lignocellulosic composition, the mechanical refining step can be controlled and tailored such that the adequate energy is used in the process. The mechanical refining may be adapted to compensate for inadequate pretreatment (when needed) in a sophisticated manner, whereby only the necessary energy is used in the process. Such controlled pretreatment also allows for savings with respect to the amount of saccharification enzymes to be used during enzymatic hydrolysis (step e). The method of the present disclosure thus allows for large savings, both with respect to costs and energy.

Accordingly, the pretreatment step may be adapted to avoid, or significantly reduce, the amount of harmful by-products, including i.a. pseudo-lignin, and the mechanical refinement may then be tailored to compensate for the suboptimal pretreatment and to disintegrate the suboptimally treated lignocellulosic composition.

In embodiments, the step of controlling the mechanical refining comprises controlling at least one parameter of the mechanical refining; the parameter being selected from:
- the energy input, specific edge load or the electrical power to be used during mechanical refining,
- the residence time in the mechanical refiner, or
- the feed rate or consistency of the first slurry into the mechanical refiner.

Mechanical refining is an energy-intense process that typically requires high amounts of electrical power, and the process is associated with high costs.

By controlling a parameter relating to the mechanical refining in response to a data set that reflects the degree, or the "severity" of the pretreatment, the subsequent mechanical refining step may be adapted and adjusted to meet the situation-specific requirements based on both the type of lignocellulosic starting material, but also based on the composition and characteristics when such starting material has been subject to suboptimal pretreatment. As mentioned, the mechanical refining process is dependent on the lignocellulosic material used. For instance, spruce may require a more severe disintegration/refinement than e.g. pine.

Optimizing and tailoring the mechanical refining process can lead to significant energy saving, lower expenses and less pollution. Furthermore, and importantly, controlling the mechanical refining step allows for a more controlled and efficient overall pretreatment process. The enzymatic hydrolysis is improved, as a more situation-specific pretreatment process allows for improved quality and stability of the pretreated slurry entering the hydrolysis. This allows for reducing and more efficiently adjusting the amount of hydrolytic enzymes used during hydrolysis.

Preferably, the parameters to be controlled are selected from the energy input, specific edge load or the electrical power used during mechanical refining.

"Specific edge load" refers to the refining intensity and may be defined as the amount of energy applied across one meter of a refiner plate's bar edge per second.

As mentioned, the composition, characteristics and size of the ingoing lignocellulosic material varies greatly, and the ingoing material may therefore respond differently to high vs. low energy intensity. The analysis step thus provides for valuable insight towards how to best optimize the mechanical refining for a specific material and a specific situation.

Furthermore, the mechanical refining and efficiency of the pretreatment largely affects the cellulose-to-sugar conversion yield.

The term "cellulose-to-sugar conversion yield" or "sugar yield", as used herein, means the fraction of sugar monomers and oligomers following hydrolysis relative to the lignocellulose material entering the hydrolysis after the pretreatment and mechanical refining steps.

In embodiments, the step(s) of analyzing the suboptimally pretreated lignocellulosic composition comprises:
- evaluating the amount of sugar and/or sugar degradation biproducts,
- evaluating the proportion between different sugars and/or sugar biproducts,
- evaluating the amount or presence of pseudo-lignin formed, or
- evaluating the size, shape and/or degree of darkness of the components of the first, suboptimally pretreated composition

These parameters reflect the degree of pretreatment, and may be used as means to control, adjust and optimize the mechanical refining for various types of lignocellulosic materials.

The amount of sugar and/or sugar biodegradation sugar typically involves measuring the total concentration of monomeric sugars. This way, information on the degree, or severity, of the pretreatment can be obtained which has an impact on how to control the subsequent mechanical refining step for a specific lignocellulosic material. A high concentration of sugar indicates a high severity of the pretreatment, whereas a low concentration indicates a low severity. It should however be noted that a too harsh pretreatment can result in reduced sugar levels due to sugar being degraded and destroyed in the process.

The proportion between different sugars and/or sugar biproducts in the pretreated lignocellulosic composition may also reflect the pretreatment degree and be analyzed to control the mechanical refining. For example, the proportion between glucose and mannose may be used as a measure of how much energy should be used during mechanical refining. The measurement of the concentration of glucose compared to the concentration of mannose is particularly suitable if softwood is used as the lignocellulosic starting material, as disclosed in EP3253892B1, assigned to Sekab E-Technology.

The amount or presence of pseudo lignin also reflects the pretreatment degree. The suboptimal pretreatment step of the present disclosure serves to reduce the amount of pseudo lignin, but in the event that pseudo lignin is formed, the mechanical refining step may be used to mechanically break it up and create more available surface for the saccharification enzymes.

Another characteristic that reflects the degree of pretreatment and may be used to control the subsequent mechanical refining is the size, shape and/or degree of darkness of the components of the suboptimally pretreated lignocellulosic composition.

As mentioned hereinbefore, lignocellulosic particles or components of smaller size are associated with higher enzymatic availability, whereas larger sized components or particles are associated with a lower enzymatic availability, the latter of which is typically linked to a suboptimal pretreatment. However, the composition of the starting materials may vary greatly, and one selected starting material may not require as much or as severe pretreatment as another. The process conditions and the energy input in the subsequent mechanical refinement may thus be tailored to meet the specific situation and the specific starting material.

The degree of darkness of the particles may correlate to enzymatic availability and improved sugar yield. Typically, darker particles correlate with higher enzymatic availability whereas brighter particles correlate with lower enzymatic availability. Furthermore, darker particles may be correlated to a higher amount of pseudo-lignin present. In the present case, brighter particles are desirable.

The shape of the components may also reflect the degree of pretreatment. As demonstrated in WO2014/114647, assigned to Sekab E-Technology, a "form factor" may be assigned to the cellulosic particles. The form factor may be a measure of how spherical or elongated the particles are. By analyzing the shape of the components, the subsequent mechanical refinement may be controlled and performed in an optimal manner.

In embodiments, the step of analyzing the components of the suboptimally pretreated lignocellulosic composition to obtain a data set reflecting the degree of the pretreatment is an automated online or inline analysis.

As used herein, "inline analysis" refers to evaluation or analysis performed inline; i.e. directly in the process line.

As used herein, "online analysis" refers to evaluation or analysis performed on a sample diverted from the process line, such as in a bypass loop from the main process line.

Automated inline and online analyses are advantageous since time consuming manual sampling methods and subsequent laboratory analyses used for controlling process parameters can be avoided. In other words, inline and online analyses provide a swift and efficient approach for correcting an ongoing process.

In embodiments, the step of analyzing the components of the suboptimally pretreated lignocellulosic composition is performed by means of an image analysis method.

The image analysis method may comprise taking a digital image in the visible range and processing the image using an algorithm such that the data set is obtained.

An image analysis method is a direct analysis method which involves extraction of meaningful information from images, in particular from digital images, by means of digital image processing techniques. The image analysis data can be used for feed-forward control the mechanical refinement.

The image analysis may be performed by means of an image analysis unit comprising an image capturing device configured to capture images in the visible, infrared (IR) or ultraviolet (UV) spectrum and an image processing device configured to process data received from the image capturing device. Data sets reflecting the degree of pretreatment can thus be obtained.

The data set may e.g. relate to information about the size, shape and/or degree of darkness of the components of the suboptimally pretreated lignocellulosic composition.

The image capturing device may be arranged to capture image(s) of cellulosic components present in the first, suboptimally pretreated lignocellulosic composition. The image(s) may be captured at a position between a pretreatment unit and the mechanical refiner or in a sample diverted from the pretreatment unit.

In embodiments, the method may comprise an additional step of analyzing the lignocellulosic particles of the second lignocellulosic composition.

If the analysis of the lignocellulosic particles of the second lignocellulosic composition reveals that the disintegration was insufficient, the second lignocellulosic composition may be subject to additional mechanical refining prior to hydrolysis.

An image analysis step may thus be included after the mechanical refinement step to analyze the lignocellulosic particles of the second lignocellulosic composition. In this case, the image analysis data can be used for feed-forward control of the enzymatic hydrolysis. For instance, the analysis of the lignocellulosic particles of the second lignocellulosic composition may indicate the amount of enzymes required during enzymatic hydrolysis.

The method is preferably based on image analysis of lignocellulosic components and particles of the suboptimally pretreated composition and correlation of at least one visual characteristic of the components to the energy, specific edge load or electrical power that is to be used in the subsequent mechanical refining. The data set generated from the analysis can be used for feedback control of the suboptimal pretreatment step and/or for feed-forward control of the mechanical refining and/or the enzymatic hydrolysis process.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### Brief description of the drawings

Figure 1 schematically illustrates the steps of a method for controlled pretreatment and/or enzymatic hydrolysis according to the present disclosure.
Figure 2 illustrates a system that may be used for a method for controlled pretreatment and/or enzymatic hydrolysis of a lignocellulosic material.

### Detailed description

The present invention will now be described more fully hereinafter with reference to the accompanying drawings. The present invention may, however, be embodied in many different forms and should not be construed as limited to the embodiments set forth herein; rather, these embodiments are provided for thoroughness and completeness, and fully convey the scope of the present invention to the skilled person.

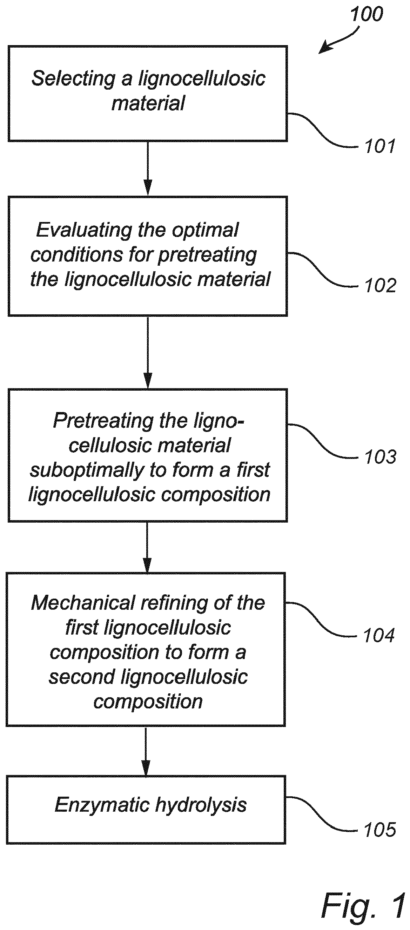

Figure 1 schematically illustrates the steps of a method for controlled pretreatment and/or enzymatic hydrolysis 100, according to the present disclosure.

First, a lignocellulosic material is selected 101. The selected material may be a specific lignocellulosic material within the category hardwood or softwood. For instance, spruce is selected.

Next, the optimal conditions for the selected lignocellulosic material, e.g. spruce, are evaluated 102. This step involves an evaluation of process parameter ranges, wherein the pretreatment is regarded as successful or optimal; i.e. yielding a high fraction of sugars after enzymatic hydrolysis. These parameters may be selected from temperature, pH, pressure or residence time in the pretreatment unit. These optimal ranges may be the result of knowledge and experience gained from a vast number of pretreatment trials for a vast number of lignocellulosic materials.

The lignocellulosic material is thereafter pretreated "suboptimally" 103; i.e. in a process parameter range outside of the range identified for the specific lignocellulosic material. A first, suboptimally pretreated lignocellulosic is thereby obtained. A suboptimal pretreatment means that the cellulose-to-sugar conversion yield is less than 50%, e.g. less than 30% of the theoretical cellulose-to-sugar conversion yield for the lignocellulosic material.

Thereafter, the first, suboptimally pretreated is subject to mechanical refining 104 to form a second lignocellulosic composition comprising lignocellulosic particles. This may be accomplished by milling or grinding.

Thereafter, the second lignocellulosic component is subject to enzymatic hydrolysis 105 to yield a hydrolysate which may be fermented and converted to a target chemical in downstream processes.

The method may comprise at least one separation step to remove pretreatment liquid from the first and/or second lignocellulosic compositions, which may also be referred to as the first and second slurries.

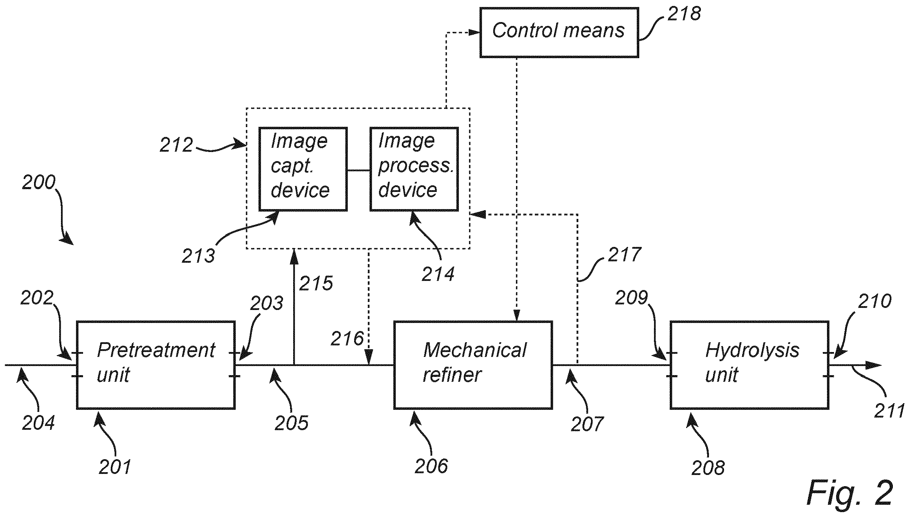

Figure 2 schematically illustrates a system 200 that may be used for a method for controlled pretreatment and/or enzymatic hydrolysis of a lignocellulosic material. The system 200 comprises a pretreatment unit 201, e.g. comprising a reactor, a vessel or a container, wherein the lignocellulosic material is pretreated suboptimally. The pretreatment unit 201 comprises an inlet 202 and an outlet 203. A feed stream of a lignocellulosic material 204 enters the pretreatment unit 201 through the inlet 202, and a first, suboptimally pretreated lignocellulosic composition 205 is discharged through the outlet 203. The suboptimally pretreated lignocellulosic composition 205 comprises a liquid component and a solid component (comprising lignin and cellulose and/or hemicellulose). The liquid component may be removed from the stream 205 in a separation unit (not shown). Such a separation unit may e.g. comprise a screen, a filter, a decanter screw, a centrifuge or similar equipment to separate and remove the liquid component from the suboptimally pretreated lignocellulosic composition 205.

Thereafter the lignocellulosic composition 205 is subject to mechanical refining in a mechanical refiner 206. The mechanical refiner 206 may be any suitable type of mill or a single or double disc refiner. Preferably, the mechanical refiner 206 is a disc refiner. The mechanical refining process may be batch refining or continuous refining.

The suboptimally pretreated lignocellulosic composition 205 is defibrated in the refiner. A second lignocellulosic composition 207 comprising lignocellulosic particles is thereby formed. The mechanical refiner 206 may comprise disc segments configured to disintegrate and/or defibrate the composition 205 to yield a second lignocellulosic composition 207. Preferably, the mechanical refiner 206 is configured to disintegrate the lignocellulosic particles such that at least 50%, such as at least 70%, preferably at least 80% of the lignocellulosic particles have a diameter in the range of from 0.1 to 500 µm, e.g. from 0.1 to 200 µm.

The mechanical refiner 206 may also comprise means for heating and/or pressurizing the lignocellulosic composition.

The system 200 further comprises an enzymatic hydrolysis unit 208 arranged downstream of and in fluid connection with the mechanical refiner 206. In the hydrolysis unit 208, the second lignocellulosic composition 207 is subject to enzymatic hydrolysis by means of saccharification enzymes. The hydrolysis unit 208 comprises an inlet 209 for receiving the second lignocellulosic composition 207, and an outlet 210 for discharge of the hydrolysate 211 after the hydrolysis has been completed.

The hydrolysate 211 may be then be subjected to separation to remove residual solids formed during hydrolysis. The hydrolysis unit 208 may be connected to a fermentation vessel and a product recovery unit (e.g. distillation or ion exchange chromatography) arranged in fluid communication with and downstream of the hydrolysis unit 208 (not shown).

The system may further comprise an image analysis unit 212 comprising an image capturing device 213 and an image processing device 214. The image capturing device 213 may comprise a light source capable of emitting light in the visible spectrum, a camera lens and a digital camera capable of capturing an image in the visible spectrum. The image processing device 214 is connected to the image capturing device 213 and is capable of processing data received from the image capturing device 213, e.g. by means of software adapted to execute image analysis.

A data set that reflects the degree of pretreatment is thereby obtained. For example, in the case of image analysis, information about components of the first, suboptimally pretreated lignocellulosic composition can be obtained such as information about the size, darkness or form of the components.

A sample 215 of the suboptimally pretreated composition 205 may be diverted from the process line and subject to image analysis prior to mechanical refining. The analyzed and/or excess sample 215 can either be discarded or returned to the enzymatic hydrolysis process through a sample return inlet 216. A sample 217 of the second lignocellulosic composition 207 may also be diverted from the process line and subject to analysis in order to control if the degree of mechanical refining was sufficient.

The system 200 further comprises means 218 for controlling the mechanical refining performed in the mechanical refiner 206 in response to the first data set so as to disintegrate the particles of the slurry further depending on how well the pretreatment has processed the lignocellulosic material. The control means 218 is preferably an automated control means, such as computer control means, suitable for online or inline control.

The means 218 for controlling the mechanical refining is arranged to communicate with the image processing device 214 of the image analysis unit 212 and with the mechanical refiner 206. The control means 218 typically receives signals, e.g. data reflecting the degree of pretreatment, from the image analysis unit 212 and outputs signals to the mechanical refiner 206 that controls the mechanical refining of the system according to predetermined control criteria. This may in embodiments involve setting the energy input, specific edge load, or the electrical power of the mechanical refiner 206, so as to optimize the the overall pretreatment. Minimal energy input (basically corresponding to no refining) may be used when the data reflecting the degree of pretreatment shows that no further disintegration in the refining step is needed. The skilled person understands that the actual control algorithms and signal handling can involve conventional control means or regulators.

The means 218 for controlling the mechanical refining may in embodiments be arranged in the same physical processing unit as the image processing device 214.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A method for controlled pretreatment and/or enzymatic hydrolysis of a lignocellulosic material comprising:
a) selecting a lignocellulosic material to be pretreated,
b) subjecting said lignocellulosic material to a suboptimal pretreatment process to form a first, suboptimally pretreated lignocellulosic composition,
c) subjecting said first, suboptimally pretreated lignocellulosic composition to mechanical refining to form a second lignocellulosic composition comprising lignocellulosic particles, and
d) subjecting said second lignocellulosic composition to enzymatic hydrolysis,
wherein said suboptimal pretreatment process is a pretreatment process, which after enzymatic hydrolysis generates a cellulose-to-sugar conversion yield of less than 50% of the theoretical cellulose-to-sugar conversion yield for said selected lignocellulosic material.

2. The method of claim 1, wherein at least 30%, e.g. at least 35%, e.g. at least 50%, e.g. at least 70% of the lignocellulosic particles of said first, suboptimally pretreated lignocellulosic composition has an average diameter above 500 µm, such as above 600 µm.

3. The method of claim 1 or claim 2, wherein said method further comprises the step of evaluating the optimal process parameters for the selected lignocellulosic material to be pretreated prior to said suboptimal pretreatment step b).

4. The method of any one of the preceding claims, wherein said suboptimal pretreatment process involves pretreating said lignocellulosic material in at least one suboptimal process parameter range, outside of an optimal process parameter range for said selected lignocellulosic material.

5. The method of claim 4, wherein said suboptimal or optimal process parameter is selected from pH, temperature, pressure or residence time in the pretreatment unit.

6. The method of claim 4 or claim 5, wherein said suboptimal or optimal process parameter is the temperature.

7. The method of any one of claims 4-6, wherein at least two suboptimal process parameter ranges are involved in said suboptimal pretreatment; said suboptimal process parameters being selected from a pH, temperature, residence time in the pretreatment unit, and pressure.

8. The method of any one of the preceding claims, wherein said suboptimal pretreatment process is a pretreatment process, which after enzymatic hydrolysis generates a cellulose-to-sugar conversion yield of less than 30% of the theoretical cellulose-to-sugar conversion yield for said lignocellulosic material

9. The method of any one of the preceding claims, wherein said suboptimal pretreatment comprises steam explosion.

10. The method of any one of the preceding claims, wherein at least 50%, such as at least 70%, preferably at least 80% of the lignocellulosic particles of said second lignocellulosic composition has an average diameter in the range of from 0.1 to 500 µm, preferably from 0.1 to 200 µm.

11. The method of any one of the preceding claims further comprising the steps of:
- analyzing said first, suboptimally pretreated lignocellulosic composition after step b) to obtain a data set reflecting the degree of said pretreatment, and
- controlling said mechanical refining of said step c) in response to said data set.

12. The method of claim 11, wherein said step of controlling said mechanical refining comprises controlling at least one parameter of said mechanical refining; said parameter being selected from:
- the energy input, specific edge load or the electrical power to be used during mechanical refining,
- the residence time in the mechanical refiner,
- the feed rate or consistency of said suboptimally pretreated composition into the mechanical refiner,

13. The method according to claim 11 or claim 12, wherein said step of analyzing said suboptimally pretreated lignocellulosic composition comprises:
- evaluating the amount of sugar and/or sugar degradation biproducts in said first, suboptimally pretreated lignocellulosic composition,
- evaluating the proportion between different sugars and/or sugar biproducts in said first, pretreated lignocellulosic composition,
- evaluating the amount of pseudo-lignin formed, or
- evaluating the size, shape and/or degree of darkness of the components of the first, suboptimally pretreated lignocellulosic composition.

14. The method of any one of claims 11-13, wherein said step of analyzing said first, suboptimally pretreated lignocellulosic composition is an automated online or inline analysis.

15. The method of any one of claims 11-14, wherein said step of analyzing said suboptimally pretreated lignocellulosic composition is performed by means of an image analysis method.
